# EUROPEAN PATENT SPECIFICATION

(11) **EP 0 489 845 B1**
(45) Date of publication and mention of the grant of the patent: **16.10.1996**
(21) Application number: 90913854.7
(22) Date of filing: 29.08.1990
(51) Int. Cl.: C07D 237/24

(54) **A METHOD OF PREPARING PYRIDAZINONE DERIVATIVES**
VERFAHREN ZUR HERSTELLUNG VON PYRIDAZINONDERIVATEN
PROCEDE DE PREPARATION DE DERIVES DE PYRIDAZINONE

(30) Priority: 30.08.1989 GB 8919622
(43) Date of publication of application: 17.06.1992
(73) Proprietor: ZENECA LIMITED, London W1Y 6LN (GB)
(72) Inventor: COX, Brian, Geoffrey 72 Clumber Road, Cheshire SK12 1NW (GB); HOWARTH, Michael, Scott 108 Church Street, Rochdale Lancashire OL15 8AS (GB)
(74) Representative: Mayall, John
(86) International application number: GB9001333
(87) International publication number: WO9103463

(56) References cited:
- EP-A- 0 049 971
- EP-A- 0 136 974
- EP-A- 0 359 474
- GB-A- 858 792
- US-A- 2 839 532
- US-A- 4 281 125

## Description

This invention relates to a method for the preparation of derivatives of pyridazinone which are useful as plant growth regulating compounds, and in particular as chemical hybridising agents. They have found use as male sterilants for cereal crops, for example wheat and barley, and are useful for making hybrids in such crops.

British Patent No. 858,792 describes the preparation of derivatives of pyridazinone which have the formula I: 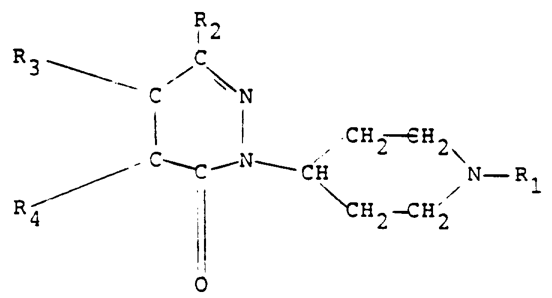 in which R₁ is an alkyl group having from 1 to 4 carbon atoms, either R₂ is an alkyl group containing 1 to 4 carbon atoms or a phenyl or thiophene residue which may be substituted, R₃ is hydrogen or an alkyl group having from 1 to 4 carbon atoms, or R₂ and R₃ together represent an alkylene chain, and R₄ is hydrogen or an alkyl, aryl or cyano group. Two procedures are described therein: the first comprises condensation of an appropriate N-alkyl-piperidyl-4-hydrazine with a γ-keto-carboxylic acid, followed by dehydrogenation of the condensation product. The second procedure involves condensing a N-alkyl-piperidyl-4-hydrazine with an α-dicarbonyl derivative to form a mono- hydrazone which is then reacted with a carboxylic acid having a reactive methylene group, or its alkyl ester.

It is known from European Patent Application No. 49971 to manufacture pyridazinone compounds according to the following reaction scheme.

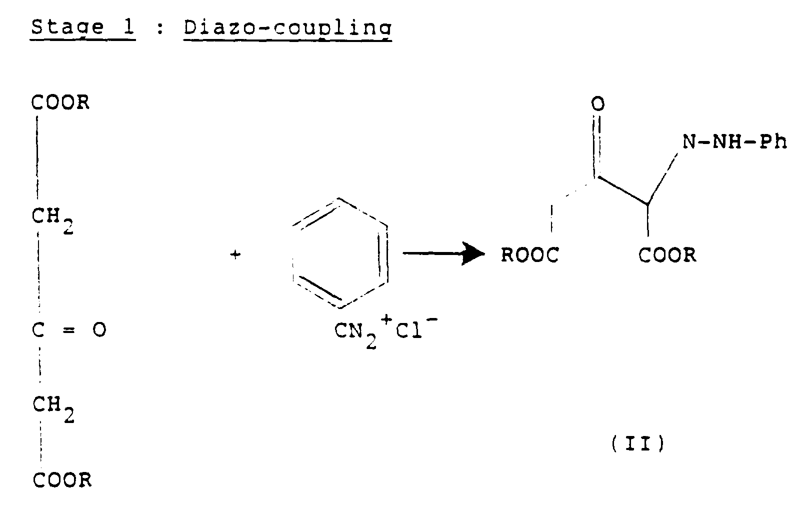

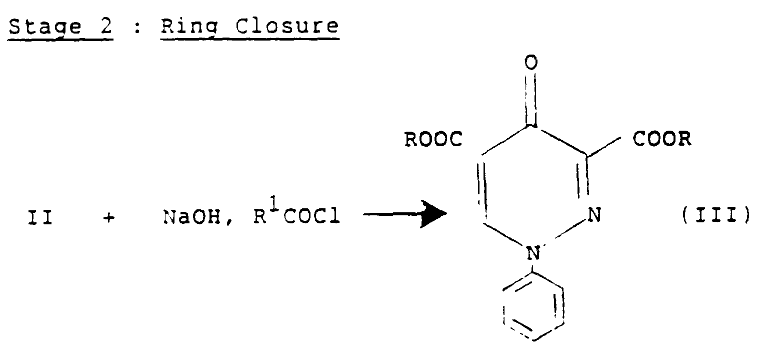

The compound (III) so obtained may then be hydrolysed either partially to the mono ester or completely to the dicarboxylic acid; and if desired, the resulting dicarboxylic acid may be partially decarboxylated. This generally gives a mixture of the two possible monocarboxylic acids, from which the desired product may be recovered. A problem with the above process is a tendency for polychlorinated biphenyls (PCBs) to be produced in Stage 1 (diazo-coupling). This is most undesirable, as PCBs are toxic and persist in the environment: accordingly considerable care and expense must be undertaken to recover them from the reaction mixture and dispose of them safely. In addition, the overall yield of the 3-carboxy derivative is 34% and no description is given in the European Application as to how the hydrolysis and decarboxylation stages necessary to obtain the 3-unsubstituted derivative has been given in the said European Application. However, one may estimate, using figures given for similar steps in am alternative procedure, that an overall yield of final product will be around 21% at best.

There has been previously proposed (British Patent Application No. 8821447.3) a method for the manufacture of 4-pyridazinone carboxylic acids of formula: 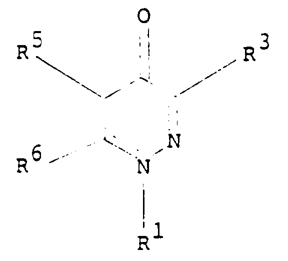 where: R¹ is a phenyl group, optionally substituted with, e.g. alkyl or halo groups, R³ is H, alkyl, halo or carboxy groups, at least one of R³ and R⁵ being carboxy; R⁵ is a carboxy group. Our method uses a novel cyclisation step which avoids Stage 1 of the scheme set out above, in which PCBs are produced in undesirable quantities, the method comprising reacting a phenylhydrazone of a glyoxylic acid halide with an enamine derivative of a ketoester. The principal disadvantages of the proposed procedure are modest yield and the tendency to formation of glyoxylic acid amides as by-products. The method may be readily understood from the following reaction scheme:

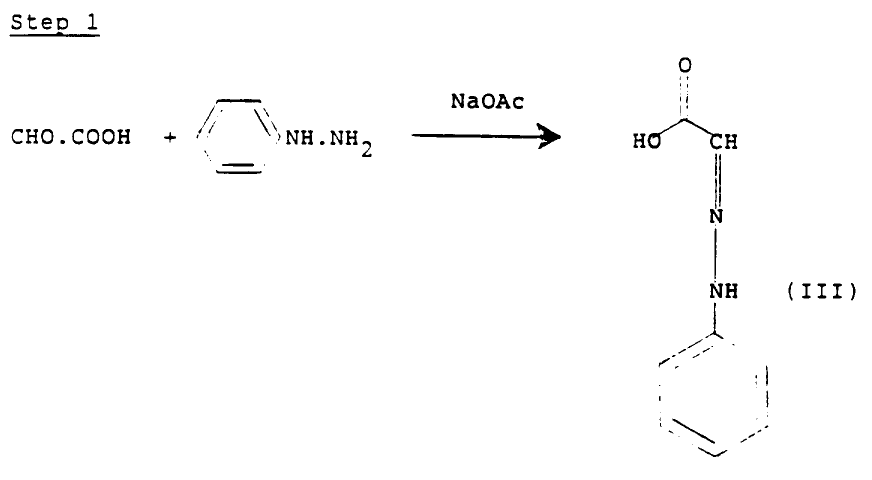

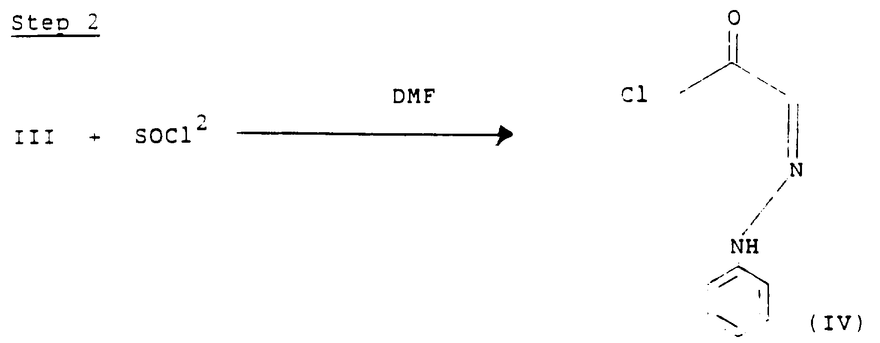

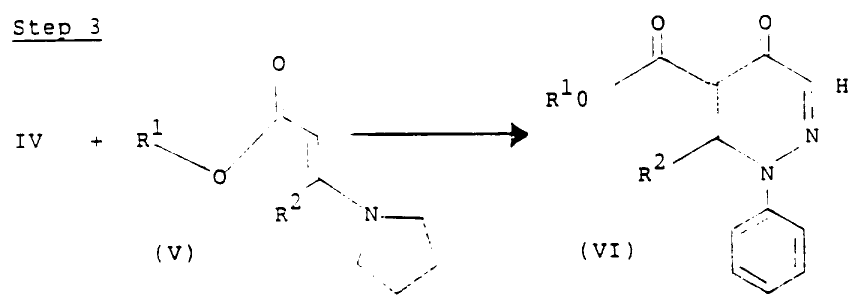

The ester (VI) so obtained may be readily hydrolysed with strong alkali to the corresponding sodium or potassium salt, a convenient form for use as a plant growth regulant or chemical hybridising agent.

An object of the present invention is to obviate or mitigate the aforesaid disadvantages.

According to the present invention there is provided a method for the preparation of a pyridazinone derivative having the general formula I: 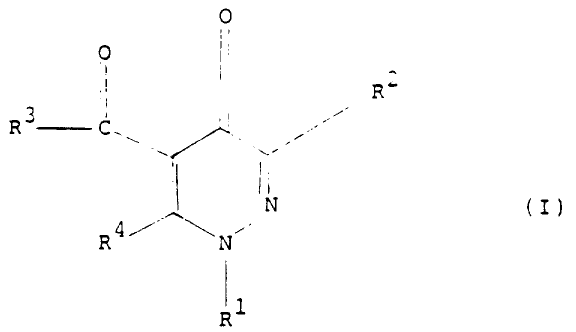 where: R¹ is a phenyl group, optionally substituted with, e.g. alkyl or halo groups, R² is H, alkyl, or carboxy groups, R³ is hydroxy or an alkyl or alkoxy group and R⁴ is an alkyl group; said method comprising reacting a hydrazone of glyoxylic acid halide having the formula VII: 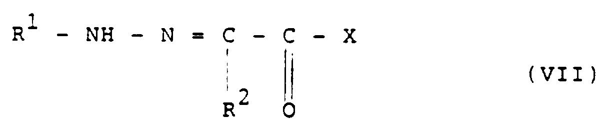 in which X represents a halogen atom and R¹ and R² are as defined above, with an oxo-carboxylic acid ester having the formula VIII; 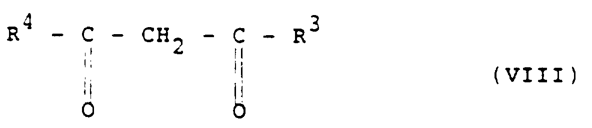 in which R³ is an alkyl or alkoxy group and R⁴ is as defined above, in the presence of an alkaline earth base. Optionally, where R³ is alkoxy, the product may then be hydrolysed to R³ = -OH.

In a modification of the method of the invention a compound of formula I may be prepared by reaction of a compound of formula VII with a preformed complex of the compound of formula VIII with the alkaline earth base.

For the purposes of this invention the term "alkaline earth base" means the oxides and hydroxides of the true alkaline earths calcium, strontium and barium, as well as of the similar element lithium.

The invention also includes the preparation of salts of the compound of general formula I above by treatment with an alkali metal compound.

In a preferred embodiment of the invention the groups R¹ to R⁴ in formula I have the following meanings:
R¹ is 4-chlorophenyl; R² is hydrogen; R³ is hydroxy or alkoxy, preferably methoxy or ethoxy; and R⁴ is ethyl.

The reaction may be conducted in a two-phase aqueous/organic system. However, it is preferred that the reaction be conducted in the presence of alkaline earth metal (particularly calcium) hydroxide in an organic liquid medium.

Figure I of the drawings shows a reaction scheme leading from p-chloroaniline to a final product compound 1-(4-chlorophenyl)-1,4-dihydro-6-ethyl-4-oxopyridazine-5-carboxylic acid. The invention will now be described, by way of illustration, in the following Examples.

### EXAMPLE 1

### (a) Preparation of the 4-chlorophenyl hydrazone of glyoxylic acid chloride

4-chlorophenyl hydrazone of glyoxylic acid (56.4g at 88% purity - 0.25 mole) was suspended in a mixture of hexane (500ml) and dimethylformamide (DMF) (3.1g). Thionyl chloride (32.8g = 0.275 mole) was added over a period of 15 minutes at ambient temperature and the mixture stirred, also at ambient temperature until gas evolution ceased. The crude product was filtered off, washed with hexane, and dried by drawing air through the product on the filter paper. The product was transferred to a beaker, methanol (100 ml) was added and the mixture stirred for about 30 seconds. The product was filtered, washed with a small amount of methanol, and dried under suction. The product was then completely dried in a desiccator.

The weight of product recovered was 52.4g with a melting point of 123-124°C. The strength by NIMR was 93.7% and the yield was 93.7%

### (b) Preparation of 1-(4-chloropheny1)-1,4-dihydro-6-ethyl-4-oxopyridazine-5-carboxylic acid

4-chlorophenyl hydrazone of glyoxylic acid chloride (10g at 97% = 0.045 mole) methyl-3-oxopentanoate (6g at 97% = 0.045 mole) and toluene (100 ml) were charged to a reaction vessel and stirred together for 5 minutes at ambient temperature. Calcium hydroxide (3.5g at 96% = 0.045 mole) was then added. The temperature rose to about 40°C. The reactants were stirred for a further 15 minutes and then 1M hydrochloric acid (100 ml) was added. The mixture was stirred for a further 5 minutes then the two phases were separated. The toluene phase was recharged to the vessel an 1M sodium hydroxide was added. The mixture was heated at 60 - 65°C for two hours then cooled and the two phases separated. The sodium hydroxide solution was added to a rapidly stirred mixture of concentrated hydrochloric acid (20 ml) and water (30 ml) at ambient temperature over about 15 minutes. The product was recovered by filtration, washed until chloride free with water and dried at 30 - 35°C and 30 mm Hg pressure. The weight of product recovered was 11.3g, representing a 70.2% yield. The strength by NMR was 76.5%.

### EXAMPLES 2-9

Further compounds of general formula IX shown below were made by the method of the invention. Structures and data for such compounds are summarised in the Table below.

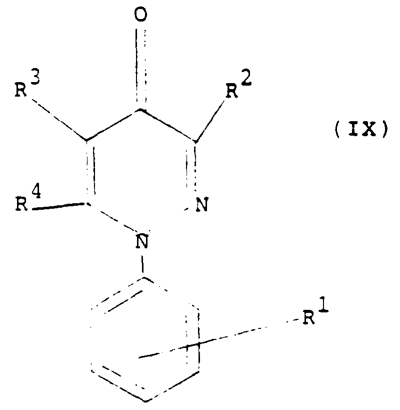

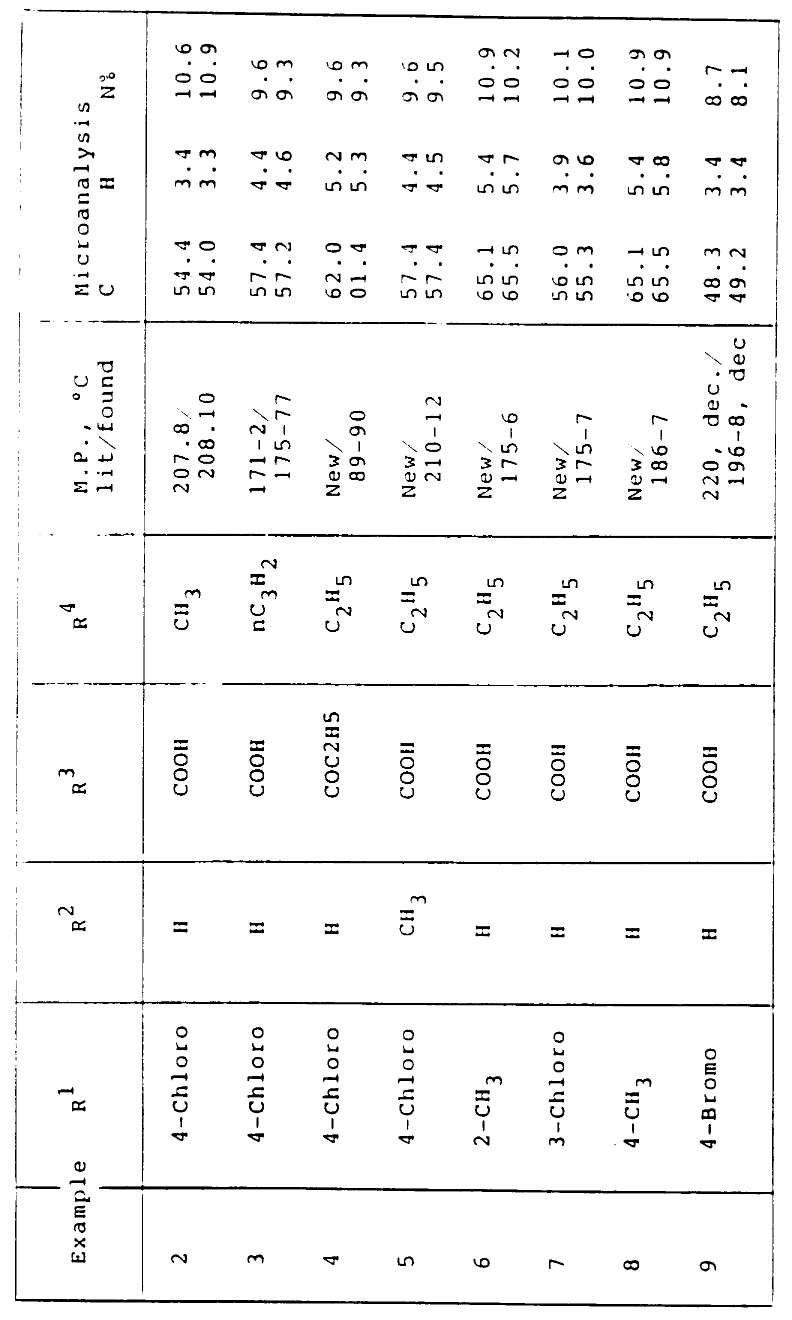

### EXAMPLE 2

### Preparation of 1-(4-chlorophenyl)-1,4-dihydro-6-methyl-4-oxo-pyridazine-5-carboxylic acid

Calcium hydroxide (4.7g at 98% = 0.062 mole) was added to a beaker containing a stirred solution of ethylacetoacetate (96.6g at 98% = 0.05 mole) and toluene (50 ml) and stirred for 2 hours at ambient temperature. The contents were then added to a reaction vessel containing 4-chlorophenyl hydrazone of glyoxylic acid chloride (11.3 g at 96% = 0.05 mole) and toluene (100mls) and stirred for 2 hours at ambient temperature. A 2 M hydrochloric acid solution (100mls) was added and stirred for 5 minutes; then the two phases were separated. The toluene phase was recharged to the vessel and heated for 2 hours at 80°C. The contents were cooled to 50°C and 10% sodium hydroxide solution (50g) was added. The mixture was then heated at 60-65°C for 3 hours then cooled and the two phases separated. The sodium hydroxide phase was added to a rapidly stirred mixture of concentrated sulphuric acid (5.9g) and water (54.1g) at ambient temperature over 1 hour. The crude product was recovered by filtration, washed with water until sulphate free and pulled fairly dry. The crude product was charged to isopropanol (25mls) and heated at reflux for 1 hour before cooling to 20°C. The purified material was recovered by filtration and washed with cold isopropanol, then dried at 40-50°C and 20 mm Hg pressure.

### EXAMPLE 3

### Preparation of 1-(4-chlorophenyl)-1,4-dihydro-4-oxo-6-n-propyl-pyridazine-5-carboxylic acid

Calcium hydroxide (4.7g at 98% = 0.062 mole) was added to a beaker containing a stirred solution of ethyl butyrylacetate (8.1g at 98% = 0.05 mole) and toluene (50ml) and stirred for 2 hours at ambient temperature. The contents were then added to a reaction vessel containing 4-chlorophenyl hydrazone of glyoxylic acid chloride (11.3g at 96% = 0.05 mole) and toluene (100mls) and stirred for 2 hours at ambient temperature. A 2M hydrochloric acid solution (100mls) was added and stirred for 5 minutes; then the two phases were separated. The toluene phase was recharged to the vessel and heated for 2 hours at 80°C. The contents were cooled at 50°C and 10% sodium hydroxide solution (50g) was added. The mixture was then heated at 60-70°C for 3 hours then cooled and the two phases separated.

The sodium hydroxide phase was added to a rapidly stirred mixture of concentrated sulphuric acid (5.9g) and water (54.1g) at ambient temperature over 1 hour. The crude product was recovered by filtration, washed with water until sulphate free and pulled fairly dry. The crude product was charged to isopropanol (25mls) and heated at reflux for 1 hour before cooling to 20°C. The purified material was recovered by filtration and washed with cold isopropanol then dried at 40-50°C and 20mm Hg pressure.

### EXAMPLE 4

### Preparation of 1-(4-chlorophenyl)-1,4-dihydro-6-ethyl-4-oxo-5-propionyl-pyridazine

Calcium hydroxide (4.7g at 98% = 0.062 mole) was added to a heater containing a stirred solution or hepta-3,5-dione (6.5g at 98% = 0.05 mole) and toluene (50ml) and stirred for 2 hours at ambient temperature. The contents were then added to a reaction vessel containing 4-chlorophenyl hydrazone of glyoxylic acid chloride (11.3g at 96% = 0.05 mole) and toluene (100mls) and stirred for 2 hours at ambient temperature. A 2M hydrochloric acid solution was added and stirred for 5 minutes then the two phases were separated. The toluene phase was recharged to the vessel and heated for 30 minutes at 80°C and then cooled to 25°C. The toluene was removed using a rotary evaporator leaving a dark brown oil. The crude solid product was crystallised from diisopyropyl ether and subsequently recrystallised using the same solvent.

### EXAMPLE 5

### Preparation of 1-(4-chlorophenyl)-1,4-dihydro-6-ethyl-3-methyl-4-oxo-pyridazine-5-carboxylic acid

4-Chlorophenyl hydrazone of pyruvic acid (11.0g at 97% = 0.05 mole), dimethyl formamide (0.6g) and n-hexane (100ml) were charged to a reaction vessel and thionyl chloride (6.5g = 0.055 mole) was added over 5 minutes with stirring at ambient temperature. The contents were stirred for 2 hours at ambient temperature before decanting off the hexane and replacing it with toluene (100ml). A calcium hydroxide/ methyl-3-oxopentanoate complex, made by stirring together methyl-3-oxopentanoate (6.6g at 98% = 0.05 mole), calcium hydroxide (4.7g at 98% = 0.062 mole) and toluene (50ml) for 2 hours at ambient temperature was added to the reaction vessel and the contents were stirred for 2 hours at ambient temperature. A 2 M hydrochloric acid solution (100mls) was added and stirred for 5 minutes; then the two phases were separated. The toluene phase was recharged to the vessel and heated at 80°C for 2 hours. The contents were cooled to 50°C and 10% sodium hydroxide solution (50g) was added. The mixture was then heated at 60-70°C for 3 hours, cooled, and the two phases separated. The sodium hydroxide phase was added to a rapidly stirred mixture of concentrated sulphuric acid (5.9g) and water (54.1g) at ambient temperature over 1 hour. The crude product was recovered by filtration, washed with water until sulphate free and pulled fairly dry.

the crude product was charged to isopropanol (25mls) and heated at reflux for 1 hour before cooling to 20°C. The purified product was recovered by filtration and washed with cold isopropanol then dried at 40-50°C and 20mm Hg pressure.

### EXAMPLE 6

### Preparation of 1-(2-methylphenyl)-1,4-dihydro-6-ethyl-4-oxo-pyridazine-5-carboxylic acid

2-Methylphenyl hydrazone of glyoxylic acid (9.2g at 97% = 0.05 mole), dimethyl formamide (0.06g) and n-hexane (100ml) were charged to a reaction vessel and thionyl chloride (6.5g = 0.055 mole) was added over 5 minutes with stirring at ambient temperature. The contents were stirred for 2 hours at ambient temperature before decanting off the hexane and replacing it with toluene (100ml). A calcium hydroxide/methyl-3-oxopentanoate complex, made by stirring together methyl-3-oxopentanoate (6.6g at 98% = 0.05 mole), calcium hydroxide (4.7g at 98% = 0.062 mole) and toluene (50ml) for 2 hours at ambient temperature, was added to the reaction vessel and the contents were stirred for 2 hours at ambient temperature. A 2M hydrochloric acid solution (100mls) was added and stirred for 5 minutes then the two phases were separated. The toluene phase was recharged to the vessel and heated at 80°C for 2 hours. The contents were cooled at 50°C and 10% sodium hydroxide solution (50g) was added. The mixture was then heated at 60-70°C for 3 hours then cooled and the two phases separated. The sodium hydroxide phase was added to a rapidly stirred mixture of concentrated sulphuric acid (5.8g) and water (54.1g) at ambient temperature over 1 hour.

The crude product was recovered by filtration, washed with water until sulphate free and pulled fairly dry. The crude product was charged to isopropanol (25ml) and heated at reflux for 1 hour before cooling to 20°C. The purified product was recovered by filtration and washed with cold isopropanol then dried at 40-50°C and 20mm Hg pressure.

### EXAMPLE 7

### Preparation of 1-(3-chlorophenyl)-1,4-dihydro-6-ethyl-4-oxo-pyridazine-5-carboxlic acid

3-Chlorophenyl hydrazone of glyoxalic acid (10.2g at 97% = 0.05 mole), dimethyl formamide (0.06g) and n-hexane (100ml) were charged to a reaction vessel and thionyl chloride (6.5g = 0.055 mole) was added over 5 minutes with stirring at ambient temperature. The contents were stirred for 2 hours at ambient temperature before decanting off the hexane and replacing it with toluene (100mls). The calcium hydroxide/methyl-3-oxopentanoate complex made by stirring together methyl-3-oxopentanoate (6.6g at 98% = 0.05 mole), calcium hydroxide (4.7g at 98% = 0.062 mole) and toluene (50ml) for 2 hours at ambient temperature was added to the reaction vessel and the contents were stirred at ambient temperature for 2 hours. A 2M hydrochloric acid solution (100mls) was added and stirred for 5 minutes; then the two phases were separated. The toluene phase was recharged to the vessel and heated for 2 hours at 80°C. The contents were cooled to 50°C and 10% sodium hydroxide solution (50g) was added. The mixture was then heated at 60-70°C for 3 hours, then cooled and the two phases separated. The sodium hydroxide phase was added to a rapidly stirred mixture of concentrated sulphuric acid (5.9g) and water (54.1g) at ambient temperature over 1 hour.

The crude product was recovered by filtration, washed with water until sulphate free and pulled fairly dry. It was then charged to isopropanol (25ml) and heated at reflux for 1 hour before cooling to 20°C. The purified material was recovered by filtration and washed with cold isopropanol then dried at 40-50°C and 20mm Hg pressure.

### EXAMPLE 8

### Preparation of 1-(4-methylphenvl)-1,4-dihydro-6-ethyl-4-oxo-pyridazine-5-carboxylic acid

4-Methylphenyl hydrazone of glyoxylic acid (9.2g at 97% = 0.05 mole), dimethyl formamide (0.06g) and n-hexane (100ml) were charged to a reaction vessel and thionyl chloride (6.5g = 0.055 mole) was added over 5 minutes with stirring at ambient temperature. The contents were stirred for 2 hours at ambient temperature before decanting off the hexane and replacing it with toluene (100ml). A calcium hydroxide/methyl-3-oxopentanoate complex made by stirring together methyl-3-oxopentanoate (6.6g at 98% = 0.05 mole), calcium hydroxide (4.7g at 98% = 0.062 mole) and toluene (50ml) for 2 hours at ambient temperature was added to the reaction vessel and the contents were stirred for 2 hours at ambient temperature. A 2M hydrochloric acid solution (100ml) was added and stirred for 5 minutes then the two phases were separated. The toluene phase was recharged to the vessel and heated at 80°C for 2 hours. The contents were cooled at 50°C and 10% sodium hydroxide solution (50g) was added. The mixture was then heated at 60-70°C for 3 hours then cooled and the two phases separated. The sodium hydroxide phase was added to a rapidly stirred mixture of concentrated sulphuric acid (5.9g) and water (54.1g) at ambient temperature over 1 hour. The crude product was recovered by filtration, washed with water until sulphate free and pulled fairly dry. The crude product was charged to isopropanol (25ml) and heated at reflux for 1 hour before cooling to 20°C. The purified product was recovered by filtration and washed with cold isopropanol then dried at 40-50°C and 20mm Hg pressure.

### EXAMPLE 9

### Preparation of -1-(4-bromophenyl)-1,4-dihydro-6-ethyl-4-oxo-pyridazine-5-carboxylic acid

4-Bromophenylhydrazone of glyoxylic acid (12.6g at 97% = 0.05 mole), dimethyl formamide (0.6g) and n-hexane (100ml) were charged to a reaction vessel and thionyl chloride (6.5g = 0.055 mole) was added over 5 minutes with stirring at ambient temperature. The contents were stirred for 2 hours at ambient temperature before decanting off the hexane and replacing it with toluene (100ml). A calcium hydroxide/methyl-3-oxopentanoate complex made by stirring together methyl-3-oxopentanoate (6.6g at 98% = 0.05 mole), calcium hydroxide (4.7g at 98% = 0.062 mole) and toluene (50ml) for 2 hours at ambient temperature was added to the reaction vessel and the contents were stirred for 2 hours at ambient temperature. A 2 M hydrochloric acid solution (100ml) was added and stirred for 5 minutes then the two phases were separated. The toluene phase was recharged to the vessel and heated at 80°C for 2 hours. The contents were cooled to 50°C and 10% sodium hydroxide solution (50g) was added. The mixture was then heated at 60-70°C for 3 hours then cooled and the two phases separated. The sodium hydroxide phase was added to a rapidly stirred mixture of concentrated sulphuric acid (5.9g) and water (54.1g) at ambient temperature over 1 hour.

The crude product was recovered by filtration and washed with water until sulphate free then pulled fairly dry. It was then charged to isopropanol (25ml) and heated at reflux for 1 hour before cooling to 20°C. The purified product was recovered by filtration and washed with cold isopropanol then dried at 40-50°C and 20mm Hg pressure.

### EXAMPLE 10

This Example illustrates the use of different alkaline earth bases in the invention to make the compound 1-(4-chlorophenyl)-1,4-dihydro-6-ethyl-4-oxo-pyridazine-5-carboxylic acid, methyl ester.

The acid chloride of 4-chlorophenylhydrazone (10g, 0.046M) and methyl-3-oxopentanoate (10.14g, 0.078M) were mixed together in toluene (100 mls). Alkaline earth metal hydroxide (0.046M) was added and the mixture stirred as the reaction was followed by HPLC. When the reaction was seen to be complete the reaction mixture was treated with 1M hydrochloric acid (95 mls) and after stirring for a short period the aqueous layer was separated ant the organic layer heated to 80°C for one hour. The toluene solution was then analysed by HPLC for the concentration of the desired product and the yield calculated. The yields for the different alkaline earth metal hydroxides are shown below.

| Base | Yield, % |
|---|---|
| Calcium hydroxide | 72.3 |
| Barium hydroxide | 43.6 |
| Lithium hydroxide | 47.1 |

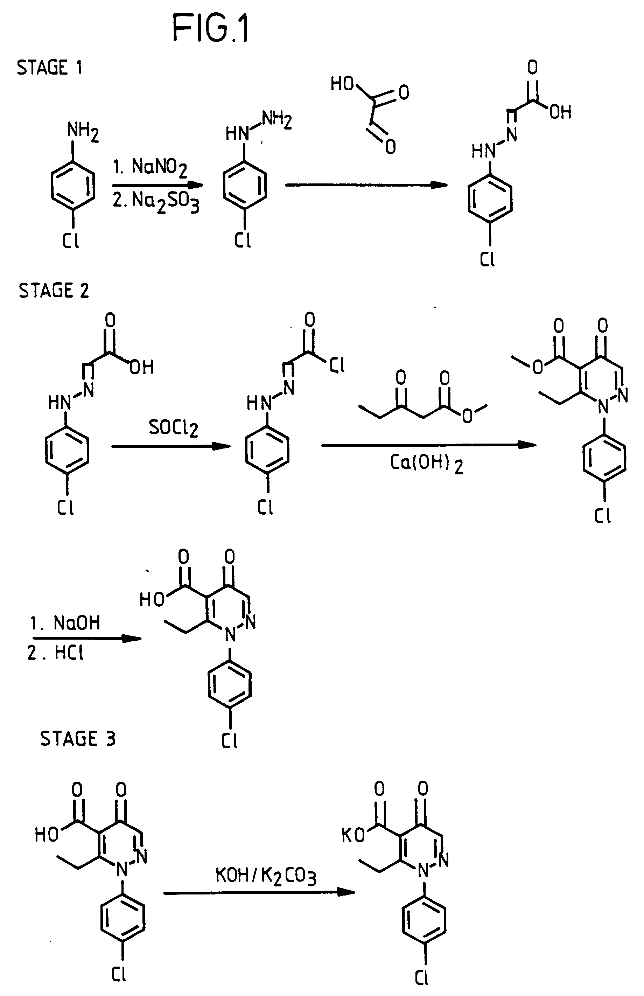

## Claims

1. The method for preparing a pyridazinone derivative having the general formula I 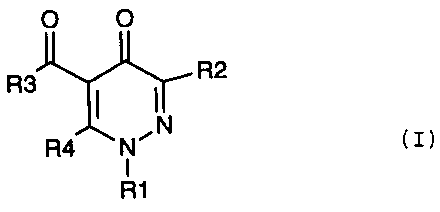 where: R¹ is a phenyl group, an alkylphenyl group or a halophenyl group, R² is H,
alkyl, or carboxy groups, R³ is hydroxy or an alkyl or alkoxy group and R⁴ is an alkyl group; characterised by reacting a hydrazone of glyoxylic acid halide having the formula VII: 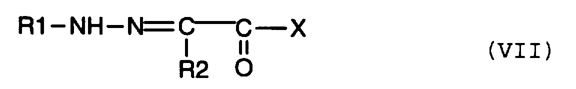 in which X represents a halogen atom and R¹ and R² are as defined above, with an oxo-carboxylic acid ester having the formula VIII; 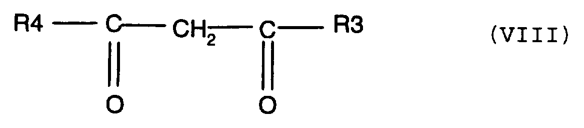 in which R³ is an alkyl or alkoxy group and R⁴ is as defined above, in the presence of an alkaline earth base selected from the oxides and hydroxides of the metals lithium, calcium, strontium and barium.

2. Method as claimed in claim 1 characterised in that the oxo-carboxylic acid of formula (VIII) is reacted with the alkaline earth base to give a preformed complex, and thereafter contacted with the hydrazone of formula (VII).

3. Method as claimed in either of claims 1 or 2 characterised in that R³ in Formula VIII is alkoxy, and the product is subsequently hydrolysed to a compound in which R³ is OH.

4. Method as claimed in any of claims 1-3 characterised in that the alkaline earth base is calcium hydroxide.

5. Method as claimed in any of claims 1-3 characterised in that the alkaline earth base is lithium oxide or hydroxide.

6. Method as claimed in any of claims 1 to 5 characterised by being carried out in an organic liquid medium.

7. Method as claimed in any of claims 1 to 6 characterised in that: R¹ is 4-chlorophenyl; R² is hydrogen; R³ is hydroxy or alkoxy; and R⁴ is ethyl.

8. Method as claimed in claim 7 characterised in that R³ is methoxy or ethoxy.

## Patentansprüche

1. Verfahren zur Herstellung eines Pyridazinon-Derivats mit der allgemeinen Formel I: 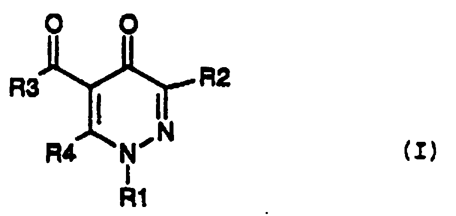 in der R¹ für eine Phenyl-Gruppe, eine Alkylphenyl-Gruppe oder eine Halogenphenyl-Gruppe steht, R² für H, Alkyl- oder Carboxy-Gruppen steht, R³ für Hydroxy oder eine Alkyl- oder Alkoxy-Gruppe steht und R⁴ für eine Alkyl-Gruppe steht, dadurch gekennzeichnet, daß ein Hydrazon eines Glyoxylsäurehalogenids mit der Formel VII: 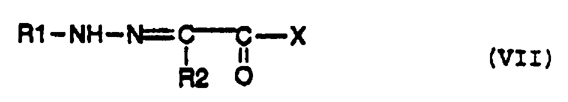 in der X ein Halogen-Atom darstellt und R¹ und R² das bereits oben definierte bedeuten, mit einem Oxocarbonsäureester mit der Formel VIII: 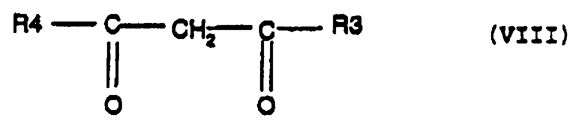 in der R³ für eine Alkyl- oder Alkoxy-Gruppe steht, in Gegenwart einer Erdalkalibase, die unter den Oxiden und Hydroxiden der Metalle Lithium, Calcium, Strontium und Barium ausgewählt ist, umgesetzt wird.

2. Verfahren nach Anspruch 1, dadurch gekennzeichnet, daß die Oxocarbonsäure mit der Formel (VIII) mit der Erdalkalibase unter Erhalt eines vorgebildeten Komplexes umgesetzt und danach mit dem Hydrazon mit der Formel (VII) in Kontakt gebracht wird.

3. Verfahren nach Anspruch 1 oder 2, dadurch gekennzeichnet, daß R³ in Formel VIII für Alkoxy steht und das Produkt anschließend zu einer Verbindung hydrolysiert wird, in der R³ für OH steht.

4. Verfahren nach einem der Ansprüche 1 bis 3, dadurch gekennzeichnet, daß die Erdalkalibase Calciumhydroxid ist.

5. Verfahren nach einem der Ansprüche 1 bis 3, dadurch gekennzeichnet, daß die Erdalkalibase Lithiumoxid oder -hydroxid ist.

6. Verfahren nach einem der Ansprüche 1 bis 5, dadurch gekennzeichnet, daß es in einem flüssigen organischen Medium durchgeführt wird.

7. Verfahren nach einem der Ansprüche 1 bis 6, dadurch gekennzeichnet, daß R¹ für 4-Chlorphenyl steht, R² für Wasserstoff steht, R³ für Hydroxy oder Alkoxy steht und R⁴ für Ethyl steht.

8. Verfahren nach Anspruch 7, dadurch gekennzeichnet, daß R³ für Methoxy oder Ethoxy steht.

## Revendications

1. Procédé de production d'un dérivé de pyridazinone répondant à la formule générale I : 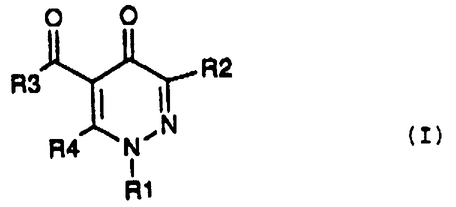 dans laquelle : R¹ est un groupe phényle, un groupe alkylphényle ou un groupe halogénophényle ; R² est de l'hydrogène, un groupe alkyle ou un groupe carboxy, R³ est un groupe hydroxy ou un groupe alkyle ou alkoxy et R⁴ est un groupe alkyle ; caractérisé par la réaction d'une hydrazone d'halogénure d'acide glyoxylique répondant à la formule VII : 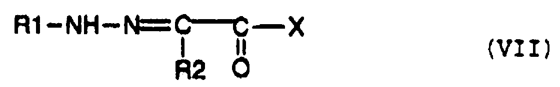 dans laquelle X représente un atome d'halogène et R¹ et R² sont tels que définis ci-dessus, avec un ester d'acide oxocarboxylique répondant à la formule VIII : 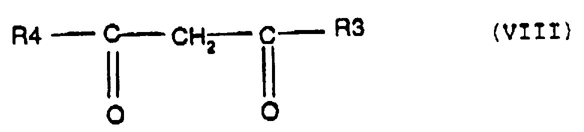 dans laquelle R³ est un groupe alkyle ou alkoxy, en présence d'une base alcalino-terreuse choisie entre des oxydes et des hydroxydes, des métaux lithium, calcium, strontium et baryum.

2. Procédé suivant la revendication 1, caractérisé en ce que l'acide oxo-carboxylique de formule VIII est amené à réagir avec la base alcalino-terreuse pour produire un complexe préformé, après quoi le complexe est mis en contact avec l'hydrazone de formule (VII).

3. Procédé suivant la revendication 1 ou 2, caractérisé en ce que R³ dans la formule VIII est un groupe alkoxy, et le produit est ensuite hydrolysé en un composé dans lequel R³ est un groupe OH.

4. Procédé suivant l'une quelconque des revendications 1 à 3, caractérisé en ce que la base alcalino-terreuse est l'hydroxyde de calcium.

5. Procédé suivant l'une quelconque des revendications 1 à 3, caractérisé en ce que la base alcalino-terreuse est l'oxyde ou l'hydroxyde de lithium.

6. Procédé suivant l'une quelconque des revendications 1 à 5, caractérisé en ce qu'il est mis en oeuvre dans un milieu liquide organique.

7. Procédé suivant l'une quelconque des revendications 1 à 6, caractérisé en ce que R¹ est le groupe 4-chlorophényle, R² est l'hydrogène ; R³ est le groupe hydroxy ou un groupe alkoxy ; et R⁴ est un groupe éthyle.

8. Procédé suivant la revendication 7, caractérisé en ce que R³ est le groupe méthoxy ou éthoxy.
